# EUROPEAN PATENT SPECIFICATION

(11) **EP 0 661 068 B1**
(45) Date of publication and mention of the grant of the patent: **01.04.1998**
(21) Application number: 94309425.0
(22) Date of filing: 16.12.1994
(51) Int. Cl.: A61M 5/32

(54) **Simplified safety syringe with retractable self-biased needle for intravenous injection**
Vereinfachte Sicherheitsspritze mit zurückziehbarer selbsttätig vorgespannter Nadel für intravenöse Injektion
Seringue de sécurité avec aiguille rétractable précontrainte pour injection intraveineuse

(30) Priority: 20.12.1993 US 169062
(43) Date of publication of application: 05.07.1995
(73) Proprietor: Chen, Long-Hsiung, Taipei (TW)
(72) Inventor: Chen, Long-Hsiung, Taipei (TW)
(74) Representative: Alexander, Thomas Bruce

(56) References cited:
- EP-A- 0 201 611
- EP-A- 0 321 903
- US-A- 5 152 750
- US-A- 5 242 402

## Description

The present invention relates to a safety syringe.

U. S. Patent 5,242,402 entitled "Safety Syringe with Retractable Self-biased Needle Adapted for Intravenous Injection" issued to the same inventor of this application disclosed a needle eccentrically secured in a plug 13 embedded in a front portion of the syringe having a needle head portion formed on a rear portion of the needle and a plunger slidably held in the syringe having an annular groove formed as a biasing socket of its longitudinal section recessed in a front portion of the plunger engageable with the needle head portion for biasing the needle obliquely within the syringe when retracting the plunger and the needle into the syringe, thereby preventing an outward protruding of the retracted needle for preventing its injury to the surroundings.

However, the plug 13 should be provided in a front portion of the syringe for holding the needle 2 in the plug 13 to be normally positioned at a front portion of the syringe 1 for injection use, and the annular groove 32 should be annularly recessed in the plunger, thereby increasing production cost and assembly inconvenience.

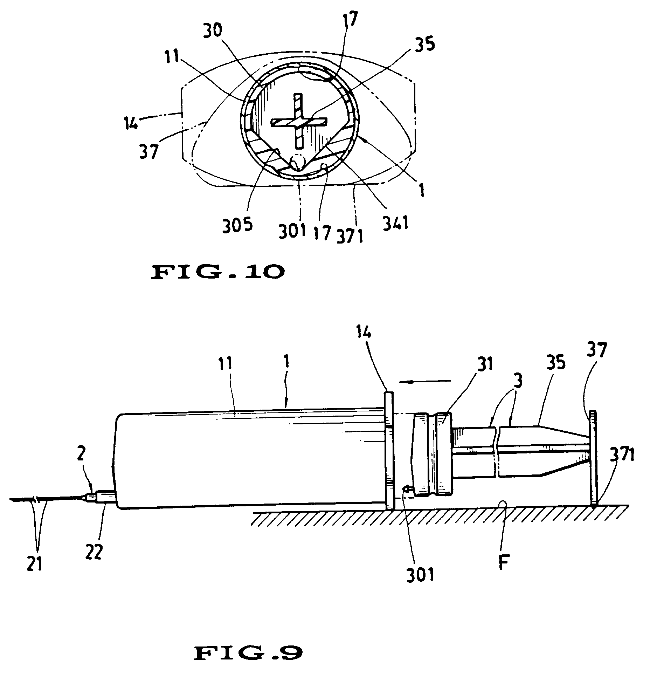

In order to save material cost and enhance assembly convenience of such a safety syringe, the plug 13 is expected to be eliminated or omitted, and the needle 2 would then be directly secured at a front portion of the syringe 1 for simplifying its production and decreasing its cost. EP-A-0 321 903 discloses in the embodiment of figure 9 a safety syringe where the needle is directly secured at a front portion of the syringe. Another embodiment which corresponds to the closest prior art is disclosed by figure 13.

According to the present invention, there is provided a safety syringe according to claim 1.

The present invention will be further described with reference to the accompanying drawings, in which:
Figure 1 is an illustration showing the present invention ready for injection use;
Figure 2 shows coupling of the plunger with the needle when finishing a medical injection;
Figure 3 shows retraction of needle into the syringe of the present invention;
Figure 4 shows a bent needle in the syringe in accordance with the present invention;
Figure 5 is an illustration showing the needle of the present invention;
Figure 6 shows a coupling member coupled with a rear needle portion in accordance with the present invention;
Figure 7 shows an inclined needle when retracted in the syringe of the present invention;

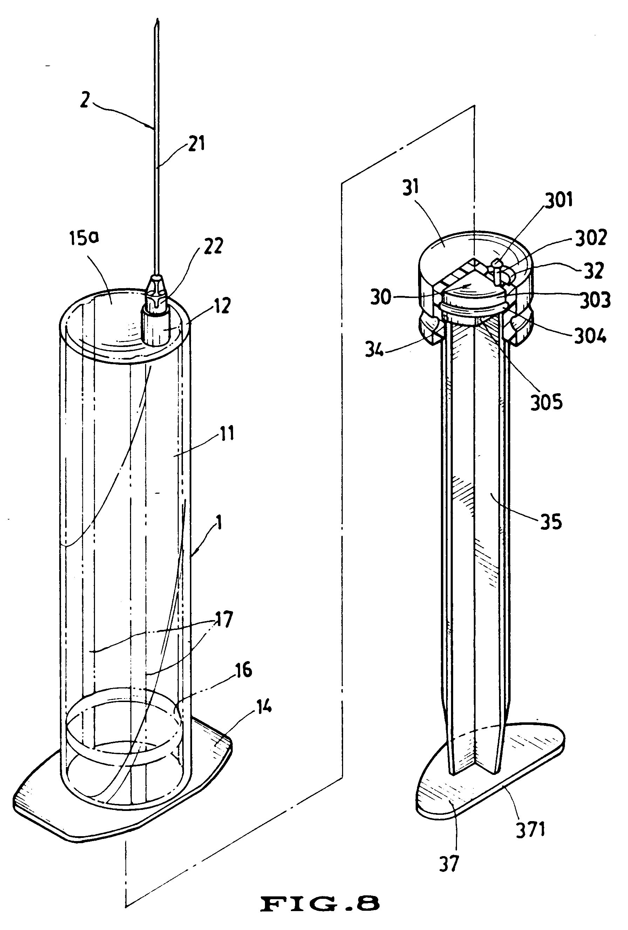

Figure 8 is a perspective view of the present invention before being assembled;
Figure 9 shows an assembly of the present invention;
Figure 10 is a sectional drawing as viewed from direction 10 - 10 of Figure 2; and
Figure 11 shows a suction tube inserted on the syringe of the present invention.

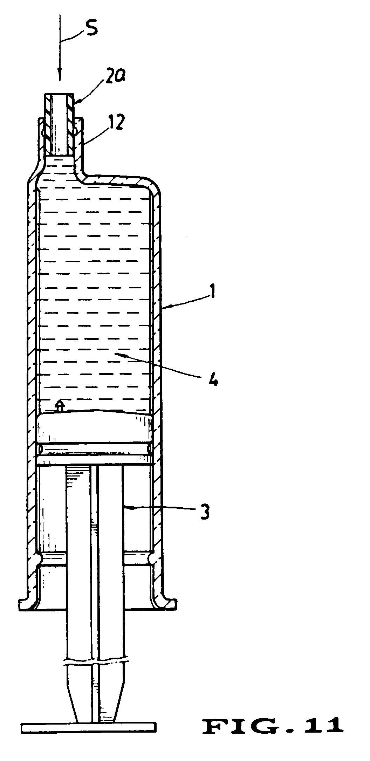

As shown in the drawing figures, the present invention comprises: a syringe means 1, a needle device 2, a plunger means 3, and optionally according to claim 2 a suction tube 2a replaceably inserted in the syringe means 1 (Figure 11).

The syringe means 1 includes: a syringe cylinder 11 having a hollow bore portion 10 defined in the syringe cylinder 11 for filling liquid medicine 4 in the cylinder 11 and a syringe axis 100 longitudinally existing in a central portion of the syringe cylinder 11, a sleeve portion 12 eccentrically formed on a front side portion of the syringe cylinder 11 contracted forwardly from the cylinder 11 having a central opening 121 formed through the sleeve portion 12, and a diverging port 122 formed in a rear portion of the sleeve portion 12 adjacent to the syringe cylinder 11, a ring groove 120 annularly recessed in a front portion of the sleeve portion 12, a syringe handle 14 formed on a rear end portion of the cylinder 11, a blocking shoulder portion 15a formed at a rear end portion of the sleeve portion 12 and positioned between the central opening 121 of the sleeve portion 12 and the bore portion 10 of the syringe cylinder 11, an annular extension 16 annularly formed on a rear portion of the cylinder 11, and a plurality of longitudinal rail extensions 17 longitudinally formed on an inside wall of the syringe cylinder 11.

The needle device 2 includes: a needle portion 21 having a needle tip 211 formed at a front end of the needle portion 21, a shank portion 22 connected with the needle portion 21 having an annular packing ring 26 circumferentially disposed around the shank portion 22 to be engageably held in the ring groove 120 in the sleeve portion 12, a bifurcated slot 25 longitudinally formed in a rear portion of the shank portion 22 and recessed forwardly from a rear needle end portion 230, at least a biasing socket 27 generally conical shaped formed in a rear portion of the shank portion 22 and communicating with a guiding port 231 recessed forwardly from the rear needle end portion 230, and a needle axis 200 longitudinally existing in a central portion of the needle device 2, with the shank portion 22 and the rear needle end portion 230 made of resilient plastic materials.

The biasing socket 27 may be formed with plural sockets, such as two sockets 27, 27a, juxtapositionally recessed in the rear needle portion of the needle device 2, which are not limited in this invention.

The needle axis 200 is parallel to the syringe axis 100 when the needle device 2 is normally secured on a sleeve portion 12 of the syringe means 1 for injection purpose.

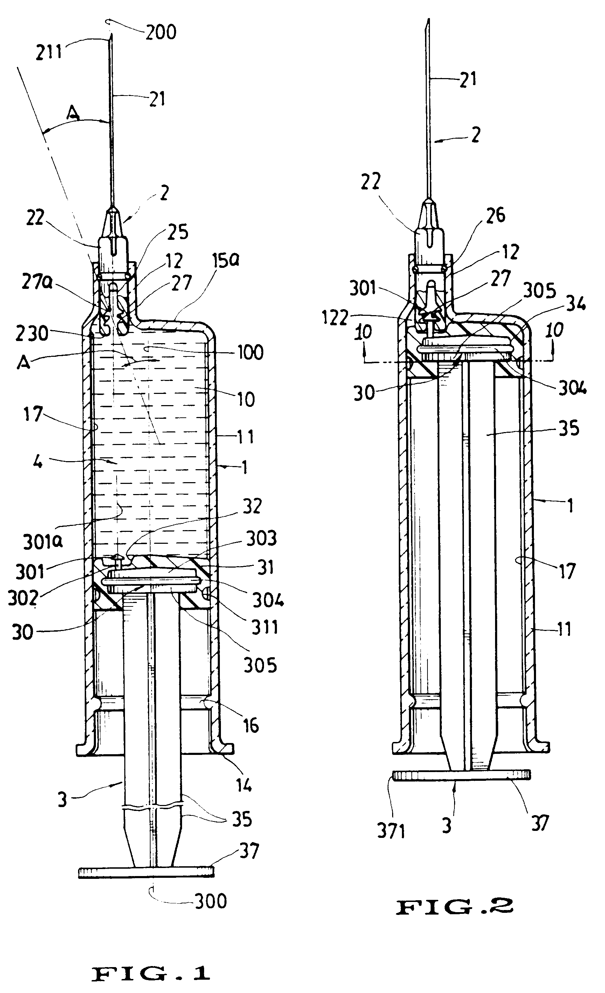

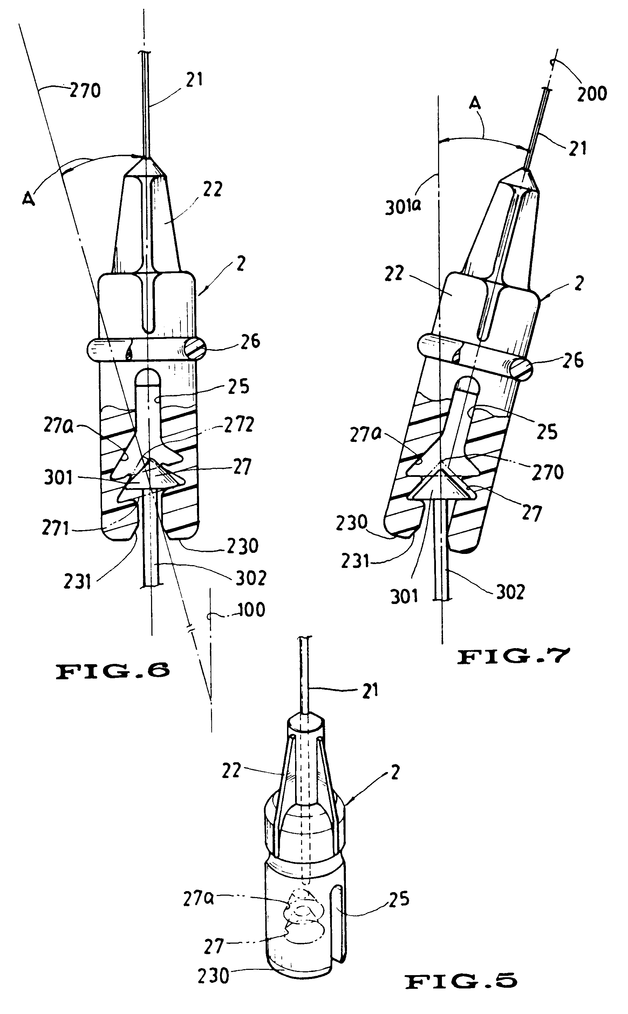

Each biasing socket 27 generally conical shaped includes: a bottom 271, an apex 272 tapered forwardly from the bottom 271, and a longitudinal axis 270 aligned with the apex 272 to be perpendicular to the bottom 271 and to be outwardly inclinedly deviated from the needle axis 200 of the needle device 2 to define an acute angle A between the needle axis 200 and the longitudinal axis 270 of the biasing socket 27. The axis 270 is eccentrically deviated from the syringe axis 100 and interpolatively intersects the syringe axis 100 with the same acute angle A as shown in Figures 1 and 6. The biasing socket 27 is snugly engageable with an arrowhead portion 301 of the plunger means 3 for obliquely biasing the needle device 2 when coupled to the plunger means 3 and retracted in the syringe cylinder 11 after finishing an injection.

The plunger means 3 includes: a plunger 31 slidably held in the syringe cylinder 11 of the syringe means 3, a coupling member 30 retained in a coupling-member recess 34 in the plunger 31 having the arrowhead portion 301 eccentrically formed on a front end of the coupling member 30 operatively insertable in a biasing socket 27 formed in the needle device 2, a holding socket 32 concentrically disposed around the arrowhead portion 301 for operatively coupling a rear needle end portion 230 when bifurcated by the arrowhead portion 301 inserted into the biasing socket 27, with the rear needle end portion 230 confined within a diverging port 122 formed in a rear portion of a sleeve portion 12 of the syringe means 1, a plunger rod 35 having a plunger handle 37 protruding rearwardly from the plunger 31 for pushing operation of the plunger 31 with the plunger 31 formed with an annular recess 311 in the plunger 31 to be engaged with an annular extension 16 formed in a rear portion of the syringe cylinder 11 for restricting a rear movement of the plunger 31, and a plunger axis 300 longitudinally defined in a central portion of the syringe means 3 normally parallel to a needle axis 200 of the needle device 2 and also parallel to an arrowhead axis 301a of the arrowhead portion 301, and aligned with the syringe axis 100 of the syringe means 1.

The coupling member 30 includes: the arrowhead portion 301 being conical shaped and engageable with a conical shaped biasing socket 27 of the needle device 2 having the arrowhead axis 301a of the arrowhead portion 301 aligned with the needle axis 200 and parallel to the plunger axis 300 and parallel to the syringe axis 100 as shown in Figure 1 ready for a normal medical injection, a neck portion 302 connected with the arrowhead portion 301, a base portion 303 having an annular protrusion 304 circumferentially formed on a periphery of the base portion 303 for well sealable embedding of the base portion 303 in the recess 34 in the plunger 31, and a secant block portion 305 engaged with a secant recess 341 formed at a rear end of the coupling-member recess 34 with the secant block portion 305 secured to a plunger rod 35 of the plunger means 3.

The plunger handle 37 has a flat bottom edge 371 slidably laid on a flat surface F as shown in Figure 9 for a straight movement of the plunger means 3 in the syringe means 1 for sharply aiming the arrowhead portion 301 at the biasing socket 27 of the needle device 2 as shown in Figures 9, 10 and 2 since the plunger 31 will not be rotated as slidably engageable with the plural longitudinal rail extensions 17 formed in the syringe cylinder 11. The secant block portion 305 engaged with the secant recess 341 also enforce such a straight forward movement of the plunger 31 in the cylinder 11.

When using the present invention for injection use as shown in Figure 1, the plunger 31 may be pushed forwardly to boost the medicine 4 in the cylinder 11 through the needle device 2 to a patient's body for an intravenous injection by tangentially inserting the eccentrically positioned needle device 2 to the patient's blood ressel.

The arrowhead portion 301 of the coupling member 30 will then be forcibly inserted into the biasing socket 27 of the needle device 2 to squeeze, and expansively bifurcate the rear needle portion 230 of the needle device 2 to store a resilient potential energy of the bifucated rear needle portion, thereby operatively coupling the coupling member 30 with the needle device 2 as shown in Figures 2, 6.

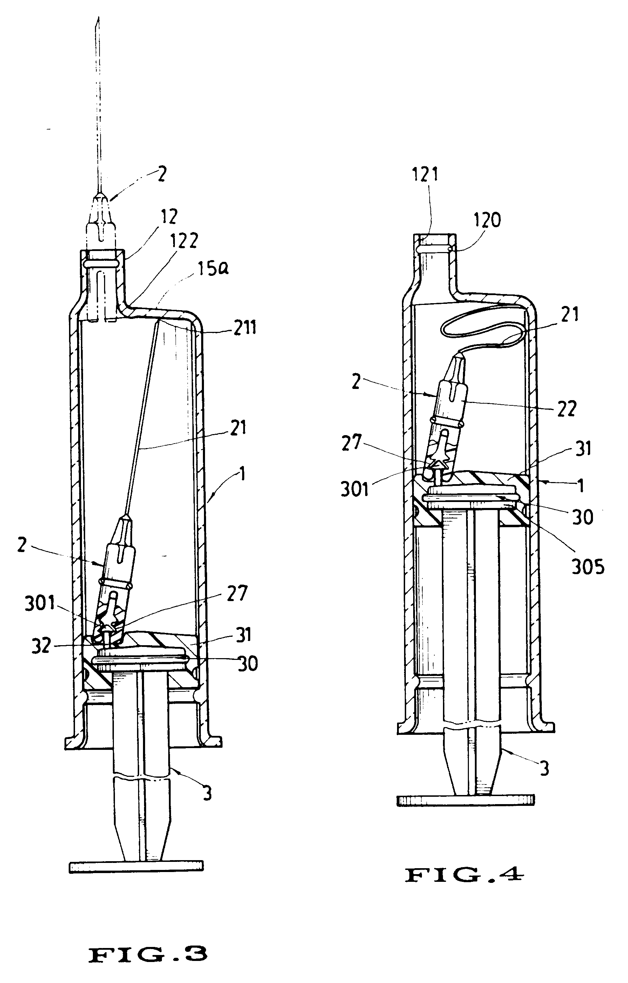

After retracting the plunger 31 and the coupled needle device 2 into the bore portion 10 of the syringe cylinder 11 as shown in Figure 3, the biasing socket 27 of the needle device 2 will be restored to be snugly engaged with the arrowhead portion 301 of the coupling member 30 of the plunger means 3 by releasing a resilient force accumulated on the rear needle portion 230 when forcibly coupling the arrowhead portion 301 with the biasing socket 27 as shown in Figures 2 and 6, thereby automatically obliquely biasing the needle device 2 coupled on the plunger 31 to intersect the syringe axis 100 and orienting towards the blocking shoulder portion 15a as shown in Figures 7 and 3. After re-protruding the needle device 2 outwardly, the needle tip 211 will be retarded against the blocking shoulder portion 15a formed in a front portion of the cylinder 11 (Figure 4), thereby bending the needle 2 and obstructing its outward protrusion and preventing its injury or contamination to the surroundings.

The present invention is superior to the earlier invention, U. S. Patent 5,242,402 also issued to the same inventor of this application since the plug 13 of the earlier invention has been eliminated, thereby saving cost and enhancing installation convenience.

Meanwhile, the needle device 2 can be conveniently replaced with diversified sizes (number of needles) directly by withdrawing a used needle 2 from the sleeve portion 12 and then by re-inserting a new needle into the sleeve portion 12 through a front opening end of the sleeve portion 12.

As shown in Figure 11, a suction tube 2a having an inside diameter larger than that of the hollow needle portion 21 may be inserted in the sleeve portion 12 for easier suction (S) of liquid medicine 4 from an ampoule (not shown). Then, the suction tube 2a may be replaced with the needle device 2 of this invention for a normal injection use.

## Claims

1. A safety syringe comprising:
a syringe means (1) including a syringe cylinder (11) having a hollow bore portion (10) for filling liquid medicine (4) therein, a sleeve portion (12) eccentrically formed on a front portion of said syringe means (1) having a central opening (121) formed through the sleeve portion (12) and a plurality of longitudinal rail extensions (17) longitudinally formed inside the syringe cylinder (11), a syringe axis (100) longitudinally defined in a central portion of said syringe means (1);
a needle device (2) including: a needle portion (21), a shank portion (22) connected with the needle portion (21) and engageably held in the sleeve portion (12), a bifurcated slot (25) longitudinally formed in a rear portion of the shank portion (22) and recessed forwardly from a rear needle end portion (230), at least a biasing-socket (27) generally conical shaped formed in a rear portion of the shank portion (22) and communicating with a guiding port (231) recessed forwardly from the rear needle end portion (230), and a needle axis (200) longitudinally defined in a central portion of the needle device (2), with the shank portion (22) and the rear needle end portion (230) made of resilient plastic materials; each said biasing socket (27) generally conical shaped including: a bottom (271), an apex (272) tapered forwardly from the bottom (271), and a longitudinal axis (270) aligned with the apex (272) to be perpendicular to the bottom (271) and to be outwardly inclinedly deviated from the needle axis (200) of the needle device (2) to define an acute angle between the needle axis (200) and the longitudinal axis (270) of the biasing socket (27); and
a plunger means (3) including: a plunger (31) slidably engageable with said plurality of longitudinal rail extensions (17) formed in said syringe cylinder (11) of the syringe means (1), a coupling member (30) retained in a coupling-member recess (34) in the plunger (31) having an arrowhead portion (301) formed on a front end of the coupling member (30) operatively insertable in said biasing socket (27) formed in the needle device (2), a holding socket (32) concentrically disposed around the arrowhead portion (301) for operatively coupling a rear needle end portion (230) when bifurcated by the arrowhead portion (301) inserted into the biasing socket (27), with the rear needle end portion (230) confined within a diverging port (122) formed in a rear portion of a sleeve portion (12) of the syringe means (1), a plunger rod (35) having a plunger handle (37) protruding rearwardly from the plunger (31) for pushing operation of the plunger (31) with the plunger (31) formed with an annular recess (311) in the plunger (31) to be engaged with an annular extension (16) formed on a rear portion of the syringe cylinder (11) for restricting a rear movement of the plunger (31), and a plunger axis (300) longitudinally defined in a central portion of the syringe means (3) normally parallel to a needle axis (200) of the needle device (2), and aligned with the syringe axis (100) of the syringe means (1); and said coupling member (30) including: the arrowhead portion (301) being conical shaped and having an arrowhead axis (301a) of said arrowhead portion (301) aligned with the needle axis (200) and parallel to the plunger axis (300) and parallel to the syringe axis (100) ready for a normal medical injection with said arrowhead portion (301) engageable with said biasing socket (27) in said needle device (2) for obliquely biasing the needle device (2) when coupled to the plunger means (3) and retracted in the syringe cylinder (11) after finishing an injection, a neck portion (302) connected with the arrowhead portion (301), a base portion (303) having an annular protrusion (304) circumferentially formed on a periphery of the base portion (303) for well sealable embedding of the base portion (303) in the coupling-member recess (34) in the plunger (31), and a secant block portion (305) engaged with a secant recess (341) of the coupling-member recess (34) and secured to a plunger rod (35) of the plunger means (3).

2. A safety syringe according to Claim 1 in combination with a suction tube (2a) having an inside diameter larger than an inside diameter of said needle portion (21) of said needle device (2), wherein said sleeve portion (12) is insertably engaged with said suction tube (2a) for easy suction of liquid medicine (4) into said syringe cylinder (11) from an ampoule, said suction tube (2a) being replaceable by said needle device (2) and said needle device (2) insertable in said sleeve portion (12) for injection use.

## Patentansprüche

1. Sicherheitsspritze, umfassend:
ein Spritzenmittel (1), umfassend einen Spritzenzylinder (11) mit einem Hohlbohrungsabschnitt (10), um flüssige Medizin (4) einzufüllen, einen Rohrstutzenabschnitt (12), der an einem vorderen Abschnitt des Spritzenmittels (1) exzentrisch ausgebildet ist und eine zentrale Öffnung (121) aufweist, die durch den Rohrstutzenabschnitt (12) ausgebildet ist, und eine Mehrzahl von Schienenlängsfortsätzen (17), die in Längsrichtung in dem Spritzenzylinder (11) ausgebildet sind und eine Spritzenachse (100), die in Längsrichtung in einem Mittelabschnitt des Spritzenmittels (1) definiert ist;
eine Nadelvorrichtung (2), umfassend:
einen Nadelabschnitt (21), einen Schaftabschnitt (22) der mit dem Nadelabschnitt (21) verbunden ist und in dem Rohrstutzenabschnitt (12) einrückbar gehalten ist, einen verzweigten Schlitz (25), der in einem hinteren Abschnitt des Schaftabschnitts (22) in Längsrichtung ausgebildet ist und vor einem hinteren Nadelendabschnitt (230) ausgespart ist, wenigstens einen Vorspannsockel (27), der in einem hinteren Abschnitt des Schaftabschnitts (22) im wesentlichen konisch geformt ausgebildet ist und mit einer Führungsöffnung (231) in Verbindung steht, die vor dem hinteren Nadelendabschnitt (230) ausgespart ist und eine Nadelachse (200), die in einem Mittelabschnitt der Nadelvorrichtung (2) in Längsrichtung definiert ist, wobei der Schaftabschnitt (22) und der hintere Nadelendabschnitt (230) aus elastischen Kunststoffmaterialien hergestellt sind;
wobei jeder im wesentlichen konisch geformte Vorspannsockel (27) umfaßt:
einen Konusboden (271), eine Konusspitze (272), die sich vom Konusboden (271) nach vorne hin verjüngt und eine Konuslängsachse (270), die nach der Konusspitze (272) ausgerichtet ist, um zum Konusboden (271) orthogonal zu sein und um nach außen geneigt von der Nadelachse (200) der Nadelvorrichtung (2) abzuweichen, um einen spitzen Winkel zwischen der Nadelachse (200) und der Konuslängsachse (270) des Vorspannsockels (27) zu definieren; und ein Kolbenmittel (3), umfassend:
einen Kolben (31), der mit der Mehrzahl von Schienenlängsfortsätzen (17) gleitend einrückbar ist, die in dem Spritzenzylinder (11) des Spritzenmittels (1) ausgebildet sind, ein Kupplungselement (30), das in einer Kupplungselementausnehmung (34) in dem Kolben (31) gehalten ist, der den Pfeilspitzenabschnitt (301) aufweist, der an einem Vorderende des Kupplungselements (30) ausgebildet ist und betriebsmäßig in den Vorspannsockel (27) einfügbar ist, der in der Nadelvorrichtung (2) ausgebildet ist, einen Haltesockel (32), der konzentrisch um den Pfeilspitzenabschnitt (301) herum angeordnet ist, um einen hinteren Nadelendabschnitt (230) betriebsmäßig zu kuppeln, wenn er durch den Pfeilspitzenabschnitt (301), der in den Vorspannsockel (27) eingefügt ist, gegabelt ist, wobei der hintere Nadelendabschnitt (230) in einer divergierenden Öffnung (122) gehalten ist, die in einem hinteren Abschnitt eines Rohrstutzenabschnitts (12) des Spritzenmittels (1) ausgebildet ist, eine Kolbenstange (35) mit einem von dem Kolben (31) nach hinten abstehenden Kolbengriff (37) zur Druckbetätigung des Kolben (31), wobei der Kolben (31) mit einer ringförmigen Ausnehmung (311) in dem Kolben (31) ausgebildet ist, um von einem ringförmigen Fortsatz (16) eingegriffen zu werden, der an einem hinteren Abschnitt des Spritzenzylinders (11) zur Beschränkung einer Rückbewegung des Kolbens (31) ausgebildet ist, und eine Kolbenachse (300), die in Längsrichtung in einem Mittelabschnitt des Spritzenmittels (3) normalerweise parallel zu einer Nadelachse (200) der Spritzenvorrichtung (2) definiert ist und nach der Spritzenachse (100) des Spritzenmittels (1) ausgerichtet ist; und wobei das Kupplungselement (30) umfaßt:
den Pfeilspitzenabschnitt (301), der konisch geformt ist und eine Pfeilspitzenachse (301a) von dem Pfeilspitzenabschnitt (301) aufweist, die nach der Nadelachse (200) ausgerichtet ist und zu der Kolbenachse (300) und zu der Spritzenachse (100) parallel ist, bereit für eine normale medizinische Injektion, wobei der Pfeilspitzenabschnitt (301) mit dem Vorspannsockel (27) in der Nadelvorrichtung (2) einkuppelbar ist, um die Nadelvorrichtung (2) schief vorzuspannen, wenn sie mit dem Kolbenmittel (3) gekuppelt ist und nach Vollendung einer Injektion in den Spritzenzylinders (11) eingezogen wird, einen Halsabschnitt (302), der mit dem Pfeilspitzenabschnitt (301) verbunden ist, einen Basisabschnitt (303), der einen ringförmigen Vorsprung (304) aufweist, der in Umfangsrichtung an einem Umfang des Basisabschnitts (303) zur gut dichtfähigen Einbettung des Basisabschnitts (303) in der Kupplungselementausnehmung (34) in dem Kolben (31) ausgebildet ist und einen Sekantenblockabschnitt (305), der mit einer Sekantenausnehmung (341) der Kupplungselementausnehmung (34) gekuppelt ist und der an einer Kolbenstange (35) des Kolbenmittels (3) befestigt ist.

2. Sicherheitspritze gemäß Anspruch 1, wobei der Rohrstutzenabschnitt (12) mit einem Saugrohr (2a) einfügbar gekuppelt ist, das einen Innendurchmesser aufweist, der größer als ein Innendurchmesser des Nadelabschnitts (21) von der Nadelvorrichtung (2) ist, um von einer Ampulle flüssige Medizin (4) leicht in den Spritzenzylinder (11) zu saugen, wobei das Saugrohr (2a) durch die Nadelvorrichtung (2) ersetzbar ist und wobei die Nadelvorrichtung (2) für einen Injektionsgebrauch in den Rohrstutzenabschnitt (12) einfügbar ist.

## Revendications

1. Seringue de sécurité, comprenant :
un moyen de seringue (1) comprenant un cylindre (11) de seringue ayant une partie creuse (10) formant un trou destiné à y introduire un médicament liquide (4), une partie en manchon (12) réalisée excentriquement sur une partie antérieure du moyen de seringue (1) et comportant un trou central (121) qui est réalisé dans la partie en manchon (12) et plusieurs protubérances en forme de barres longitudinales (17) réalisées longitudinalement à l'intérieur du cylindre de seringue (11), un axe (100) de seringue situé longitudinalement dans une partie centrale du moyen de seringue (1) ;
un dispositif à aiguille (2) comprenant : une partie (21) formant une aiguille, une partie de tige (22) reliée à la partie d'aiguille (21) et tenue par appui dans la partie en manchon (12), une fente en forme de fourche (25) réalisée longitudinalement dans une partie arrière de la partie de tige (22) et comportant un évidement situé au-devant d'une partie d'extrémité arrière (230) de l'aiguille, au moins un alvéole de poussée (27) de forme sensiblement conique qui est réalisé dans une partie arrière de la partie de tige (22) et communiquant avec un orifice de guidage (231) formant un évidement à l'avant de la partie d'extrémité arrière (230) de l'aiguille, un axe (200) de l'aiguille situé longitudinalement dans une partie centrale du dispositif à aiguille (2), la partie de tige (22) et la partie d'extrémité arrière d'aiguille (230) étant réalisées en matière plastique élastique ; chacun desdits alvéoles de poussée (27) de forme sensiblement conique comprenant : une base de cône (271) , un sommet conique (272) se rétrécissant au-devant de la base de cône (271) et un axe longitudinal de conicité (270) aligné sur le sommet conique (272) de manière à être perpendiculaire à la base du cône (271) et à être décalé de manière inclinée vers l'extérieur de l'axe de l'aiguille (200) du dispositif à aiguille (2) de manière à inscrire un angle aigu entre l'axe (200) de l'aiguille et l'axe longitudinal de conicité (270) de l'alvéole de poussée (27) ; et
un moyen de piston plongeur (3) comprenant : un piston plongeur (31) en appui de coulissement contre lesdites plusieurs protubérances (17) en forme de barres longitudinaux qui sont réalisées dans ledit cylindre de seringue (11) du moyen de seringue (1), un élément d'assemblage (30) retenu dans une cavité (34) du piston plongeur (31) destinée à l'élément d'assemblage et comportant une partie (301) en pointe de flèche qui est réalisée sur une extrémité antérieure de l'élément d'assemblage (30) et qui peut être introduite en service dans ledit alvéole de poussée (27) réalisé dans le dispositif à aiguille (2), une douille de support (32) disposée concentriquement autour de la partie en pointe de flèche (301) pour raccorder fonctionnellement une partie d'extrémité arrière (230) de l'aiguille lorsqu'elle est écartée par la partie en pointe de flèche (301) introduite dans l'alvéole de poussée (27), la partie d'extrémité arrière (230) de l'aiguille étant confinée dans un orifice divergeant (122) réalisé dans une partie arrière de la partie en manchon (12) du moyen de seringue (1), une tige (35) du piston plongeur comportant une poignée (37) de piston plongeur qui est saillante à l'arrière du piston plongeur (31) pour l'opération de poussée du piston plongeur (31), le piston plongeur (31) comportant une cavité annulaire (311) réalisée dans le piston plongeur (31) et destinée à se placer sur une protubérance annulaire (16) réalisée sur une partie arrière du cylindre de seringue (11) pour restreindre un mouvement vers l'arrière du piston plongeur (31) et un axe (300) de piston plongeur qui est situé longitudinalement dans une partie centrale du moyen de seringue (3) et normalement parallèle à un axe (200) de l'aiguille du dispositif à aiguille (2) et qui est aligné sur l'axe (100) de la seringue du moyen de seringue (1) ; et ledit élément d'assemblage (30) comprenant : la partie en pointe de flèche (301) qui est en forme conique et qui comporte un axe (301a) de pointe de flèche de ladite partie (301) en pointe de flèche qui est aligné sur l'axe de l'aiguille (200) et qui est parallèle à l'axe (300) du piston plongeur et qui est parallèle à l'axe (100) de la seringue prête pour une injection médicale normale, la partie en pointe de flèche (301) pouvant se loger dans ledit alvéole de poussée (27) du dispositif à aiguille (2) pour pousser obliquement le dispositif à aiguille (2) lorsqu'il est relié au moyen de piston plongeur (3) et pour le mettre en retrait dans le cylindre de seringue (11) après achèvement d'une injection, une partie de col (302) étant liée à la partie en pointe de flèche (301), une partie de base (303) comportant une protubérance annulaire (304) réalisée à la circonférence sur une périphérie de la partie de base (303) pour noyer de manière bien étanche la partie de base (303) dans la cavité (34) du piston plongeur (31) réservée à l'élément d'assemblage et une partie sécante de bloc (305) logée dans une cavité sécante (341) de la cavité (34) réservée à l'élément d'assemblage et fixée à une tige de piston (35) du moyen de piston plongeur (3).

2. Seringue de sécurité selon la revendication 1, dans laquelle ladite partie en manchon (12) peut être introduite et être en appui dans un tube d'aspiration (2a) ayant un diamètre intérieur plus grand que le diamètre intérieur de ladite partie d'aiguille (21) dudit dispositif à aiguille (2) pour faciliter l'aspiration d'un médicament liquide (4) dans ledit cylindre de seringue (11) à partir d'une ampoule, ledit tube d'aspiration (2a) pouvant être remplacé par ledit dispositif à aiguille (2) et ledit dispositif à aiguille (2) pouvant être introduit dans ladite partie en manchon (12) pour une utilisation pour une injection.
